# EUROPEAN PATENT APPLICATION

(11) **EP 3 607 970 A1**
(43) Date of publication of application: **12.02.2020**
(21) Application number: 18780467.9
(22) Date of filing: 04.04.2018
(51) Int. Cl.: A61K 39/395, A61K 47/00, A61P 37/00

(54) **LYOPHILIZED PREPARATION OF INFLIXIMAB MONOCLONAL ANTIBODY**

(30) Priority: 07.04.2017 CN 201710224783
(71) Applicant: Hisun Bioray Pharmaceutical Co., Ltd., Taizhou, Zhejiang 318000 (CN)
(72) Inventor: FANG, Weijie, Taizhou, Zhejiang 318000 (CN); WANG, Haibin, Taizhou, Zhejiang 318000 (CN); QIAN, Ci, Taizhou, Zhejiang 318000 (CN); GAO, Dong, Taizhou, Zhejiang 318000 (CN)
(74) Representative: Schnappauf, Georg
(86) International application number: PCT/CN2018/081807
(87) International publication number: WO 2018/184541

(57) **Abstract**

Provided is a lyophilized preparation of infliximab monoclonal antibody, including infliximab, a histidine buffer system, a structural protective agent and a surfactant; after reconstitution, concentration of infliximab monoclonal antibody in said preparation is 100-200 mg/ml.

## Description

The present application claims the priority of Chinese Patent Application No. 201710224783.5, filed on April 7, 2017, entitled "Lyophilized Preparation of Infliximab monoclonal antibody". The content of which is incorporated by reference in its entirety.

### Technical Field

The present invention relates to the field of pharmacy, and in particular, to a lyophilized formulation containing infliximab. The lyophilized formulation can be reconstituted to obtain an infliximab formulation suitable for subcutaneous administration.

### Background Technique

Infliximab monoclonal antibody (infliximab, CAS # 170277-31-3) is a human-mouse chimeric monoclonal antibody that prevents the binding of TNF-α to its cell surface receptor by specifically binding to TNF-α in human, thereby blocking the biological activity of TNF-α, ultimately reduces the inflammatory response and reduces osteoclast activation, achieving the purpose of controlling and alleviating symptoms. Infliximab can be used to treat rheumatoid arthritis, psoriasis, ankylosing spondylitis, ulcerative colitis and Crohn's disease, etc.

The currently marketed Remicade® is a lyophilized formulation. During the preparation of the injection formulation, the concentration of infliximab in the system after reconstitution with water is 10 mg/mL, and the formulation is administered by intravenous injection (i.v.). Rheumatoid arthritis, psoriasis, ankylosing spondylitis, ulcerative colitis, and Crohn's disease are chronic diseases that require long-term or even life-long administration. Intravenous administration may only be carried out in a hospital environment, and usually, the administration of 100-200 ml of the product takes one hour or more, which is inconvenience for the patients, and thus the compliance with the formulation is poor on the current market.

High-concentration infliximab formulation may significantly shorten the time of administration. Due to its ease of use, high-concentration infliximab formulation has become the focus of attention. However, increasing the concentration of the monoclonal antibody will increase the viscosity of the formulation solution, making the preparation and administration of the formulation more difficult.

Therefore, a stable high-concentration biological formulation suitable for subcutaneous administration has a large market demand and prospect.

### Summary of Invention

In one aspect, provided is a lyophilized formulation comprising infliximab, histidine buffer system, structure protectant, and surfactant, wherein
before lyophilization, in the formulation, the concentration of infliximab is from about 10 to about 80 mg/ml, the concentration of histidine in the histidine buffer system is from about 1 to about 20 mM, the concentration of the structure protectant is from about 5 to about 100 mg/ml and the concentration of the surfactant is from about 0.001 to 1 mg/ml; and after reconstitution, the concentration of infliximab in the formulation is from about 100 to 200 mg/ml.

In a preferred embodiment, before lyophilization, the concentration of infliximab in the formulation may be from about 25 to about 60 mg/ml, more preferably from about 30 to about 50 mg/ml, and most preferably about 40 mg/ml.

In another preferred embodiment, before lyophilization, the concentration of histidine in the formulation may be from about 3 to about 10 mM, preferably about 5 mM.

In yet another preferred embodiment, the structure protectant in the formulation is selected from the group consisting of carbohydrates, amino acids, and combinations thereof, preferably sucrose, trehalose or combinations thereof, more preferably sucrose. Before lyophilization, the concentration of the structure protectant may be from about 5 to about 75 mg/ml, more preferably from about 5 to about 50 mg/ml, and most preferably about 25 mg/ml.

In still another preferred embodiment, the surfactant in the formulation is selected from the group consisting of polysorbates, poloxamer, triton, sodium dodecyl sulfate, sodium lauryl sulfate, sodium octyl glycoside, lauryl-sulfobetaine, polyethylene glycol, polypropylene glycol or a mixture thereof, preferably polysorbate 80. Before lyophilization, the concentration of the surfactant may be from about 0.01 to about 0.4 mg/ml, more preferably from about 0.025 to about 0.1 mg/ml.

In one embodiment, before lyophilization, the pH of the formulation is from about 4.0 to about 8.0, preferably from about 4.5 to about 6.0, more preferably from about 5.0 to about 5.4.

In one embodiment, after reconstitution of the lyophilized formulation, the reconstituted formulation has at least one of the following properties:
the viscosity is about 40 cP or less, and
the osmotic pressure is from about 250 to about 530 mOsmol/kg.

In one embodiment, after reconstitution, the concentration of infliximab in the formulation may be from about 130 to about 190 mg/ml, more preferably from about 140 to about 170 mg/ml, from about 150 to about 160 mg/ml.

In still another aspect, the invention also relates to the use of the lyophilized formulation for the manufacture of a medicament for the prevention or treatment of human autoimmune-associated diseases.

In still another aspect, the invention also relates to a method for the prevention or treatment of human autoimmune-associated diseases, the method comprising administering to a subject in need thereof a lyophilized formulation according to the invention.

In yet another aspect, the invention also relates to a lyophilized formulation according to the invention for use in the prevention or treatment of human autoimmune-associated diseases.

In one embodiment, the diseases comprise rheumatoid arthritis, ankylosing spondylitis, Crohn's disease, ulcerative colitis, psoriatic arthritis, plaque psoriasis, and the like.

### Detailed Description

### General definition and terminology

Unless otherwise stated, the terms and phrases used herein have the meanings listed below. Particular terms or phrases should not be considered as undefined or unclear when they are not specifically defined and should be interpreted according to what is generally understood by those skilled in the art. When a trade name appears in this document, it is intended to refer to its corresponding product or its active ingredient.

When used with a numerical variable, the term "approximate" or "about" generally refers to the value of the variable and all the values of the variable within the experimental error (for example, within the 95% confidence interval of the mean) or within ± 10% of the specified value, or a wider range.

The expression "comprise" or its synonyms "contain", "include", "have" or the like is open-ended, which does not exclude other unlisted elements, steps or ingredients. The expression "consist of" excludes any unlisted elements, steps or ingredients. The expression "substantially consist of" refers to specified elements, steps or ingredients within a given range, together with optional elements, steps or ingredients which do not substantively affect the basic and novel features of the claimed subject matter. It should be understood that the expression "comprise" encompasses the expressions "substantially consist of" and "consist of".

The term "optional" or "optionally" as used herein means that the subsequently described event or circumstance may or may not occur, and the description includes the occurrence or non-occurrence of the event or circumstance. For example, the composition optionally includes an antioxidant covering the case with or without an antioxidant.

The percentages, parts, and the like herein are calculated based on weight unless otherwise indicated.

The infliximab used herein is commercially available and has the CAS Number of 170277-31-3, e.g., may be obtained from Zhejiang Hisun Pharmaceutical Co., Ltd.

The term "histidine buffer" or "histidine buffer system" refers to a buffer system obtained by combining histidine with an acid which is optionally present for pH adjustment, e.g., hydrochloric acid, acetic acid, sulfuric acid and the like. When characterizing a histidine buffer system in a formulation, the sum of the concentrations of histidine, including its free and ionized forms, is typically used. Examples of histidine buffers include, but are not limited to, histidine/hydrochloric acid, histidine/acetic acid, histidine/sulfuric acid, and the like. A preferred histidine buffer of the present invention is a histidine/hydrochloric acid buffer.

The term "carbohydrate" includes monosaccharides, disaccharides, oligosaccharides, and polysaccharides. Examples of carbohydrates include, but are not limited to, fructose, glucose, sucrose, trehalose, mannose, lactose, mannose, maltose, sorbose, dextran, dextrin, cyclodextrin, hydroxyethyl starch, or combinations thereof.

The term "amino acid" includes amino acids and/or pharmaceutically acceptable salts thereof. Examples of amino acids include, but are not limited to, alanine, arginine, asparagine, aspartic acid, cysteine, glutamine, glutamic acid, glycine, histidine, isoleucine, leucine, lysine, methionine, phenylalanine, proline, serine, threonine, tryptophan, tyrosine, valine or combinations thereof.

"Pharmaceutically acceptable salt" refers to a salt formed with a pharmaceutically acceptable non-toxic base or acid, including inorganic or organic bases and inorganic or organic acids. For example, a salt formed with the following inorganic acids: for example, hydrochloric acid, hydrobromic acid, hydroiodic acid, sulfuric acid, phosphoric acid or nitric acid; or a salt formed with the following organic acids: for example, formic acid, acetic acid, acetoacetic acid, pyruvic acid, trifluoroacetic acid, propionic acid, butyric acid, caproic acid, heptanoic acid, undecanoic acid, lauric acid, benzoic acid, salicylic acid, nicotinic acid, pamoic acid, picric acid, lauryl sulfate, ethanesulfonic acid, benzenesulfonic acid, p-toluenesulfonic acid, methanesulfonic acid, citric acid, tartaric acid, stearic acid, lactic acid, oxalic acid, malonic acid, succinic acid, malic acid, alginic acid, maleic acid, fumaric acid, D-gluconic acid, mandelic acid or ascorbic acid.

The term "lyophilization" refers to a drying technique, in which a solution formulation is frozen to a solid state at a lower temperature, and then the water therein is sublimed directly to a gaseous state under vacuum without passing through a liquid state, and finally, the material is dehydrated. Lyophilization includes, but is not limited to, freezing, primary drying, and secondary drying.

In the present invention, a liquid composition is first prepared, then lyophilized to obtain the lyophilized formulation of the present invention. Thus, such liquid compositions are also referred to herein as "primary liquid". The term refers to the form before lyophilization of the lyophilized formulation. The formulation is usually carried out in an aqueous solution (usually water or physiological saline, for example, water for injection, etc.).

The term "reconstitution" or "reconstituted" means that the components of the lyophilized formulation are dissolved in an aqueous solution (for example, but not limited to, ethanol, water, aqueous dextrose or a mixture thereof, e.g., water for injection, physiological saline, etc.) to obtain a pharmaceutical formulation after reconstitution. The reconstitution of the lyophilized formulation of the present invention can be carried out by techniques well known to those skilled in the art. Therefore, the reconstituted formulation of the present invention can be obtained by reconstituting the lyophilized formulation of the present invention. The term refers to the form of the lyophilized formulation after reconstitution.

The term "osmotic pressure" herein refers to the osmotic pressure after reconstitution of the lyophilized formulation. The osmotic pressure should be suitable for subcutaneous administration. The osmotic pressure after reconstitution of the lyophilized formulation of the present invention may be from about 250 to about 530 mOsmol/kg, for example, from about 250 to about 430 mOsmol/kg, from about 300 to about 400 mOsmol/kg.

### Infliximab composition, lyophilized formulation and formulation after reconstitution

There are following problems during developing high-concentration infliximab formulation: First, during the preparation of the primary liquid before lyophilization, an increase in the concentration of the monoclonal antibody would increase the viscosity, making preparation and administration difficult. The method to reduce the viscosity by sacrificing the concentration of other components, e.g., structure protectant, is likely to make the stability of the system difficult to be maintained. The lyophilized formulations in the prior art, e.g., Remicade®, can achieve a higher concentration of infliximab in the formulation after reconstitution by adjusting the water during reconstitution, but the adjustment of water during reconstitution would increase the concentration of other components, e.g., sucrose, in the formulation. Accordingly, the osmotic pressure of the system is increased, making the administration impossible.

Second, many detection methods, such as protein quantification, are subjected to interference at high concentrations.

In addition, a formulation is more likely to denature, aggregate and precipitate under high concentrated conditions. The degraded products thereof have a major impact on the safety of biopharmaceuticals. In particular, some protein aggregates would stimulate the body's immune response, which would reduce the efficacy of biopharmaceuticals, or even cause the death of patients. For the antibody drugs of the infliximab species, not only high-purity product is required during manufacturing, but also structural stability during transportation, storage and administration should be maintained. Therefore, how to maintain the stability of the system while maintaining a high concentration of infliximab, and making viscosity and osmotic pressure suitable for processing and administration is the key to solving the problem. The range of osmotic pressure suitable for subcutaneous administration is, for example, from about 250 to about 530 mOsmol/kg, from about 250 to about 430 mOsmol/kg, from about 300 to about 400 mOsmol/kg.

The present invention relates to a lyophilized formulation comprising infliximab, a histidine buffer system, a structure protectant and a surfactant.

In one embodiment, before lyophilization, the concentration of infliximab in the primary liquid is from about 10 to about 80 mg/ml, preferably from about 25 to about 60 mg/ml, more preferably from about 30 to about 50 mg/ml, most preferably about 40 mg/ml.

In the formulation of the present invention, examples of the histidine buffer system include, but are not limited to, histidine/hydrochloric acid, histidine/acetic acid, histidine/sulfuric acid, and the like, preferably histidine/hydrochloric acid buffer. In one embodiment, before lyophilization, the concentration of histidine in the histidine buffer system of the primary liquid is from about 1 to about 20 mM, preferably from about 3 to about 10 mM, more preferably about 5 mM.

The pharmaceutical formulation of the present invention may comprise a structure protectant. The structure protectant can be used to stabilize the structure of the active ingredient in the pharmaceutical composition, providing protection at room temperature and lyophilized state. Carbohydrates and amino acids are commonly used stabilizers for protein drugs. It also acts as a cryoprotectant and a drying protecting agent to protect the protein from denaturation that may occur during lyophilization. In one embodiment, the structure protectant is carbohydrate, amino acid, or combinations thereof. In a preferred embodiment, the structure protectant is selected from the group consisting of sucrose, trehalose, arginine hydrochloride and combinations thereof, more preferably sucrose. The structure protectant of overly low concentration would reduce the stability of the formulation. In a preferred embodiment, before lyophilization, the concentration of the structure protectant in the primary liquid is from about 5 to about 100 mg/ml, preferably from about 5 to about 75 mg/ml, more preferably from about 5 to about 50 mg/ml, e.g., about 10, about 15, about 20, about 25 and about 30 mg/ml, most preferably about 25 mg/ml.

The pharmaceutical formulation of the present invention may further comprise a surfactant.

The surfactant is a substance that can change (usually reduce) the surface tension of a liquid or the interfacial tension between two phases. The surfactant is amphiphilic and contains a hydrophilic group and a lipophilic group. Examples of the surfactant include, but are not limited to, polysorbates, poloxamer, triton, sodium dodecyl sulfate, sodium lauryl sulfate, sodium octyl glycoside, lauryl-sulfobetaine, polyethylene glycol, polypropylene glycol or mixtures thereof. Examples of polysorbates include but are not limited to polysorbate 20, polysorbate 21, polysorbate 40, polysorbate 60, polysorbate 61, polysorbate 65, polysorbate 80, polysorbate 81, polysorbate 85 or a mixture thereof, preferably polysorbate 80. Before lyophilization, the concentration of the surfactant in the primary liquid is from about 0.001 to about 1 mg/ml, preferably from about 0.01 to about 0.4 mg/ml, and most preferably from about 0.025 to about 0.1 mg/ml.

The pharmaceutical formulation of the present invention may optionally contain an antioxidant which inhibits oxidation of the pharmaceutical formulation, improves the stability of the drug and contributes to prolonging the storage period. Examples of antioxidants include, but are not limited to, ascorbic acid, tryptophan, methionine, glutathione, sodium thiosulfate, catalase, or combinations thereof.

The pharmaceutical formulation of the present invention may also optionally contain a preservative, which inhibits the growth and reproduction of microorganisms, including, but not limited to, m-cresol, phenol, benzyl alcohol, benzalkonium chloride, phenoxyethanol, p-hydroxybenzoate, or combinations thereof.

The pharmaceutical formulation of the present invention may also comprise one or more other ingredients known to those skilled in the art, such as the isotonicity agent sodium chloride, the metal chelating agent EDTA, and the like.

The selection of pH is critical to the stability of the formulation and the pH should be chosen such that the corresponding active ingredient remains stable. In addition, the pH should be chosen such that other ingredients in the primary liquid retain their physical and chemical properties, e.g., without degradation, before lyophilization. As a preferred embodiment, a suitable pH is from about 4.0 to about 8.0, preferably from about 4.5 to about 6.0, more preferably from about 5.0 to about 5.4, e.g., about 5.2.

The reconstituted formulation of the present invention can be obtained by reconstitution of the lyophilized formulation of the present invention.

The concentration of infliximab in the formulation after reconstitution is from about 100 to about 200 mg/ml, preferably from about 130 to about 190 mg/ml, more preferably from about 140 to about 170 mg/ml, from about 150 to about 160 mg/ml.

The reconstituted formulation of the present invention has at least one of the following properties:
1) Viscosity: about 40 cP or less, such as about 30 cP or less, about 20 cP or less, about 10 cp or less, or about 5 cp or less; and
2) Osmotic pressure: the osmotic pressure is suitable for subcutaneous administration, or the osmotic pressure is from about 250 to about 530 mOsmol/kg, for example from about 250 to about 430 mOsmol/kg, from about 300 to about 400 mOsmol/kg.

### Beneficial effect

Infliximab lyophilized formulation of the present invention can be stable for long-term storage in a lyophilized state, and can achieve a high-concentration monoclonal antibody and a low viscosity during administration after reconstitution, and is suitable for subcutaneous administration. The primary liquid, lyophilized formulation and reconstituted formulation of the present invention solve the problems of high viscosity and poor stability that are easily occurring when infliximab has a high concentration, and have a very broad market application prospect.

### Examples

The invention is illustrated in more detail with reference to the examples. However, these examples are for illustrative purposes only and the invention is not limited by these examples.

### Preparation

According to the lyophilized formulation design scheme, the amount of addition was calculated according to the concentration of the infliximab primary liquid, volume and concentration of the components, and the drug solution to be lyophilized was prepared with water for injection. After mixing the components, sterilization was performed using a 0.22 µm Millipore sterile filtration membrane. A dispensation was conducted to 2 ml vials (West Pharmaceutical) and visual inspection was performed such that all products had qualified appearance, which were colorless or pale yellow, clear liquid, and had no visible foreign matter. The products were lyophilized, and the lyophilization process is shown in Table 1. After filling or lyophilization, a coated rubber stopper and an aluminum-plastic cover were added.

**Table 1. Infliximab lyophilization process**

| | Shelf temperature (°C) | Conversion time (min) | Maintenance time (min) | Chamber pressure |
|---|---|---|---|---|
| Prefreeze | 5 | N/A | 60 | Atmospheric pressure |
| | -40 | 100 | 90 | Atmospheric pressure |
| | -5 | 80 | 120 | Atmospheric pressure |
| | -40 | 80 | 90 | Atmospheric pressure |
| Primary drying (sublimation) | -20 | 125 | 1440 | 0.075 mbar |
| Secondary drying (desorption) | 25 | 250 | 600 | 0.075 mbar |

High temperature and light exposure experiments were conducted independently for the lyophilized formulation.

High temperature experiment: the sample was placed in a 40 °C ± 2 °C incubator, sampled and measured on Days 14 and 28, respectively;
Light exposure experiment: the sample was placed in an illumination box (5 °C ± 3 °C, 4500 Lx ± 500 Lx) and sampled 10 days later.

### Reagents and testing instruments

### Reagents:

Infliximab, provided by Zhejiang Hisun Pharmaceutical Co., Ltd.
Histidine, purchased from JT Baker
Sucrose, purchased from Merck KGaA
Trehalose, purchased from HAYASHIBARA Co., Ltd., Japan
Arginine hydrochloride, purchased from JT Baker
Polysorbate 80, purchased from JT Baker

### Instruments:

Agilent 1200 liquid chromatography, purchased from Agilent, USA.
SEC-HPLC chromatographic column: TSK-GEL G3000 SWXL from TOSOH, Japan, 7.8×300 mm; mobile phase: 0.02 mol/L sodium dihydrogen phosphate, 0.2 mol/L sodium chloride buffer, 10% acetonitrile, pH 7.0; flow rate: 0.5 ml/min; column temperature: 30 °C; running time: 30 min; wavelength: 280 nm.

IEC-HPLC column: Thermo MabPac-SCX-10 BioLC™, 4×250 mm Analytical; detection wavelength: 214 nm; mobile phase A: 10 mM sodium dihydrogen phosphate, pH 6.8; mobile phase B: 10 mM sodium dihydrogen phosphate dihydrate, 0.1 M sodium chloride, pH 6.8; flow rate: 0.8 ml/min; column temperature: 30 ° C; injection volume: 10 µl; gradient: 0 min, 10% mobile phase B; 5 min, 10% mobile phase B; 40 min, 60% mobile phase B; 42 min, 100% mobile phase B; 47 min, 100% mobile phase B; 49 min, 10% mobile phase B; 60 min, 10% mobile phase B.

Broomfield DV2T viscometer: purchased from Broomfield, USA, the method of measuring viscosity was carried out according to the manufacturer's instructions.

Vogel OM815 osmometer: purchased from Germany LOSER, the method of measuring osmotic pressure was carried out according to the manufacturer's instructions.

### Example 1. Effect of different structure protectants on the stability of infliximab

Each of the primary liquids (the initial liquid concentration of the monoclonal antibody was 40.0 mg/ml) was prepared according to Table 2, and trehalose, sucrose, and arginine hydrochloride and combinations thereof were used as protectants. The primary liquid was lyophilized, and then the high temperature and light exposure experiments were conducted for lyophilized formulations.

**Table 2. The content of each component in the primary liquid**

| Serial number | Histidine buffer (mM) | Protectant | Polysorbate 80 (mg/ml) | pH |
|---|---|---|---|---|
| Formulation 1 | 5.0 | 27.6 mg/ml dihydrate trehalose | 0.025 | 5.2 |
| Formulation 2 | 5.0 | 25.0 mg/ml sucrose | 0.025 | 5.2 |
| Formulation 3 | 5.0 | 9.0 mg/ml arginine hydrochloride | 0.025 | 5.2 |
| Formulation 4 | 5.0 | 13.8 mg/ml dihydrate trehalose, | 0.025 | 5.2 |
| | | 4.5 mg/ml arginine hydrochloride | | |
| Formulation 5 | 5.0 | 12.5 mg/ml sucrose, | 0.025 | 5.2 |
| | | 4.5 mg/ml arginine hydrochloride | | |
| Formulation 6 | 5.0 | 18.4 mg/ml dihydrate trehalose, | 0.025 | 5.2 |
| | | 3 mg/ml arginine hydrochloride | | |
| Formulation 7 | 5.0 | 16.67 mg/ml sucrose, 3 mg/ml arginine hydrochloride | 0.025 | 5.2 |

After the experiment, the content of the aggregation in the lyophilized formulation was determined by size exclusion chromatography (SEC-HPLC), the main peak content of the charge variants was measured by ion-exchange chromatography (IEC-HPLC), and the results are shown in Tables 3 and 4. The content of the aggregation is typically used to reflect the physical stability of the lyophilized formulation, and the main peak content of the charge variants is generally used to reflect the chemical stability of the lyophilized formulation.

It can be seen from Tables 3 and 4 that the content of the aggregation in the lyophilized formulation is increased after the high temperature experiment, and the main peak content of the charge variants is decreased. All lyophilized formulations show a small increase in the aggregation content after 10 days of light exposure and a small decrease in the main peak content of the charge variants, i.e., show relatively good physical and chemical stability. After the lyophilized formulation is placed at 40 °C for 14 days and 28 days, the lyophilized formulation of Formulation 2 with sucrose as a protectant has a relatively low aggregation content and a relatively high main peak content of the charge variants, i.e., has favourable physical and chemical stability.

**Table 3. The content of aggregation (%) in each of the lyophilized formulations after high temperature and light exposure**

| Serial number | Day 0 | 40 ° C for 14 days | 40 ° C for 28 days | Light exposure for 10 days |
|---|---|---|---|---|
| Formulation 1 | 0.92±0.03 | 2.50±0.15 | 3.23±0.06 | 1.06±0.05 |
| Formulation 2 | 0.91±0.00 | 1.88±0.09 | 2.52±0.03 | 1.01±0.01 |
| Formulation 3 | 0.99±0.01 | 3.43±0.09 | 4.45±0.04 | 1.31±0.02 |
| Formulation 4 | 0.98±0.01 | 2.64±0.02 | 3.57±0.04 | 1.17±0.04 |
| Formulation 5 | 0.96±0.02 | 2.34±0.02 | 3.12±0.02 | 1.13±0.05 |
| Fonnulation 6 | 0.95±0.05 | 2.49±0.21 | 3.41±0.03 | 1.12±0.03 |
| Formulation 7 | 0.90±0.03 | 2.16±0.05 | 2.89±0.05 | 1.08±0.01 |

**Table 4. The main peak content of the charge variants in each of the lyophilized fonnulations after high temperature and light exposure (%)**

| Serial number | Day 0 | 40 ° C for 14 days | 40 ° C for 28 days | Light exposure for 10 days |
|---|---|---|---|---|
| Formulation 1 | 68.63±0.38 | 64.17±1.80 | 62.30±0.15 | 67.24±0.21 |
| Formulation 2 | 69.13±0.79 | 64.92±0.26 | 64.86±0.37 | 67.75±0.82 |
| Formulation 3 | 69.06±0.35 | 62.75±0.38 | 60.19±0.92 | 67.35+0.22 |
| Formulation 4 | 69.31±0.31 | 63.79±0.67 | 62.96±0.60 | 67.49±0.15 |
| Formulation 5 | 69.65±0.54 | 64.14±0.86 | 63.30±0.81 | 67.10+0.27 |
| Formulation 6 | 69.24±0.62 | 63.88±0.90 | 62.95±1.03 | 67.29±0.50 |
| Formulation 7 | 69.36±0.40 | 63.22±0.99 | 62.98±1.23 | 67.00±0.39 |

The lyophilized formulation was reconstituted with a small amount of water (1 ml filling volume, reconstituted with 0.22 ml water), and the monoclonal antibody concentration, viscosity, and osmotic pressure were measured and the results are shown in Table 5. The content of the monoclonal antibody in the formulation after reconstitution is between 136.20 and 143.89 mg/ml, and the viscosity and osmotic pressure are suitable for subcutaneous administration.

**Table 5. Viscosity and osmotic pressure of formulations after reconstitution**

| Serial number | Infliximab (mg/ml) | Viscosity (CP) | Osmotic pressure (mOsmol/kg) |
|---|---|---|---|
| Formulation 1 | 143.89 | 22.93 | 475 |
| Formulation 2 | 143.76 | 23.91 | 451 |
| Formulation 3 | 143.76 | 34.56 | 397 |
| Formulation 4 | 138.79 | 24.77 | 424 |
| Formulation 5 | 139.82 | 24.65 | 421 |
| Formulation 6 | 136.20 | 22.5 | 438 |
| Formulation 7 | 139.19 | 22.81 | 439 |

### Example 2. Effect of different concentrations of surfactant on the stability of infliximab

Each of the primary liquids (the initial liquid concentration of infliximab was 40 mg/ml) was prepared according to Table 6, and lyophilization was performed. The lyophilized formulation was subjected to high temperature and light exposure experiments.

**Table 6. The content of each component in the primary liquid**

| Serial number | Histidine buffer (mM) | Dihydrate trehalose (mg/ml) | Polysorbate 80 (mg/ml) | pH |
|---|---|---|---|---|
| Formulation 8 | 5 | 27.6 | 0.025 | 5.2 |
| Formulation 9 | 5 | 27.6 | 0.1 | 5.2 |

The content of the aggregation of the lyophilized formulation after the experiment was determined by size exclusion chromatography (SEC-HPLC), and the main peak content of the charge variants was determined by ion-exchange chromatography (IEC-HPLC). The results are shown in Tables 7 and 8.

**Table 7. The content of aggregation (%) in each of the lyophilized formulations after high temperature and light exposure experiments**

| Serial number | Day 0 | 40 ° C for 14 days | 40 ° C for 28 days | Light exposure for 10 days |
|---|---|---|---|---|
| Formulation 8 | 0.92±0.03 | 2.50±0.15 | 3.23±0.06 | 1.06±0.05 |
| Formulation 9 | 0.92±0.02 | 2.47±0.06 | 3.37±0.02 | 1.12±0.01 |

**Table 8. The main peak content of the charge variants (%) in each of the lyophilized formulations after high temperature and light exposure experiments**

| Serial number | Day 0 | 40 ° C for 14 days | 40 ° C for 28 days | Light exposure for 10 days |
|---|---|---|---|---|
| Formulation 8 | 68.63±0.38 | 64.17±1.80 | 62.30±0.15 | 67.24±0.21 |
| Formulation 9 | 68.42±0.15 | 62.63±0.01 | 61.93±0.05 | 66.63±0.03 |

It can be found from Table 7 that different concentrations of surfactant do not have a significant effect on the increase of the aggregation content of infliximab. However, after the 40 °C experiment, the lyophilized formulation containing 0.025 mg/ml polysorbate 80 has a lower decrease of the main peak content of the charge variants than the lyophilized formulation containing 0.1 mg/ml polysorbate 80, i.e., under high temperature condition, the chemical stability of the lyophilized formulation with the content of polysorbate 80 being 0.025 mg/ml is better. In addition, the lyophilized formulation containing 0.025 mg/ml and 0.1 mg/ml polysorbate 80 both retain favourable main peak content of the charge variants under light exposure conditions, i.e., favourable chemical stability.

The lyophilized formulation was reconstituted with a small amount of water (1 ml filling volume, reconstituted with 0.22 ml water) and the concentration of the monoclonal antibody, viscosity and osmotic pressure were determined, and the results are shown in Table 9. The content of the monoclonal antibody in the formulation after reconstitution is between 141.57 and 143.89 mg/ml, and the viscosity and osmotic pressure are suitable for subcutaneous administration.

**Table 9. Viscosity and osmotic pressure for each of the formulations after reconstitution**

| Serial number | Infliximab (mg/ml) | Viscosity (CP) | Osmotic pressure (mOsmol/kg) |
|---|---|---|---|
| Formulation 8 | 143.89 | 22.93 | 475 |
| Formulation 9 | 141.57 | 22.83 | 471 |

### Example 3. Effect of different concentrations of monoclonal antibody and structure protectant and their combinations with pH on the stability of infliximab

Each of the primary liquids was prepared according to Table 10, and lyophilization was performed. The lyophilized formulation was subjected to high temperature and light exposure experiments.

**Table 10. The content of each component in the primary liquid**

| Serial number | Infliximab (mg/ml) | Histidine buffer (mM) | Sucrose (mg/ml) | Polysorbate 80 (mg/ml) | pH |
|---|---|---|---|---|---|
| Formulation 10 | 40 | 5 | 25 | 0.025 | 5.0 |
| Formulation 11 | 40 | 5 | 25 | 0.025 | 5.4 |
| Formulation 12 | 50 | 5 | 25 | 0.025 | 5.0 |
| Formulation 13 | 50 | 5 | 25 | 0.025 | 5.4 |
| Formulation 14 | 40 | 5 | 23 | 0.025 | 5.0 |
| Formulation 15 | 50 | 5 | 23 | 0.025 | 5.0 |
| Formulation 16 | 40 | 5 | 20 | 0.025 | 5.0 |

The lyophilized formulation was subjected to high temperature and light exposure experiments, and the content of the aggregation in the lyophilized formulation was determined by size exclusion chromatography (SEC-HPLC), and the main peak content of the charge variants was determined by ion-exchange chromatography (IEC-HPLC). The results are shown in Table 11 and Table 12.

It can be found from Table 11 that an increase in the concentration of the monoclonal antibody will increase the content of the aggregation in the lyophilized formulation, but do not have a significant effect on the main peak content of the charge variants. The increase in sucrose concentration is beneficial to the decrease of aggregation content but does not have significant effect on the main peak content of the charge variants. The pH of 5.0 or 5.4 does not have significant effect on the main peak content of the charge variants, but the aggregation content was higher at pH of 5.4 compared to pH of 5.0 (Formulation 10 compared to Formulation 11 or Formulation 12 compared to Formulation 13). That is, the monoclonal antibody concentration, sucrose concentration and the pH within a certain range will affect the stability of lyophilized formulation, especially the physical stability, to some extent. An increase in the concentration of the monoclonal antibody may reduce the physical stability of the lyophilized formulation, and an increase in the concentration of the sucrose may improve the physical stability of the lyophilized formulation.

**Table 11. The content of aggregation (%) of each of the lyophilized formulations after high temperature and light exposure experiments**

| Serial number | Day 0 | 40 ° C for 14 days | 40 ° C for 28 days | Light exposure for 10 days |
|---|---|---|---|---|
| Formulation 10 | 0.74±0.02 | 2.10±0.08 | 2.46±0.03 | 0.94±0.03 |
| Formulation 11 | 0.83±0.01 | 2.30±0.10 | 2.48±0.00 | 1.01±0.01 |
| Fonnulation 12 | 0.80±0.00 | 2.68±0.02 | 2.96±0.03 | 1.02±0.02 |
| Formulation 13 | 0.88±0.01 | 2.74±0.03 | 3.09±0.03 | 1.13±0.05 |
| Formulation 14 | 0.81±0.01 | 1.61±0.15 | 2.78±0.03 | 0.99±0.01 |
| Formulation 15 | 0.88±0.01 | 2.42±0.06 | 3.56±0.08 | 1.13±0.01 |
| Formulation 16 | 0.81±0.00 | 1.99±0.01 | 2.80±0.03 | 1.00±0.01 |

**Table 12. The main peak content of the charge variants (%) of each of the lyophilized formulations after high temperature and light exposure experiments**

| Serial number | Day 0 | 40 ° C for 14 days | 40 ° C for 28 days | Light exposure for 10 days |
|---|---|---|---|---|
| Formulation 10 | 72.65±1.71 | 65.86±0.66 | 64.65±0.71 | 70.40+0.98 |
| Formulation 11 | 71.67±0.71 | 66.16+0.57 | 64.45±0.07 | 69.82+0.84 |
| Formulation 12 | 72.41±0.94 | 66.42±2.78 | 63.86±0.21 | 69.30±0.21 |
| Formulation 13 | 71.97+0.55 | 65.70+1.43 | 64.13 | 69.73±0.98 |
| Formulation 14 | 70.41+0.44 | 66.57±0.18 | 64.47±0.33 | 70.25+1.14 |
| Formulation 15 | 70.14±0.65 | 65.14±0.36 | 62.85±0.33 | 68.93±0.20 |
| Formulation 16 | 70.32±0.26 | 65.74±0.34 | 63.09±0.11 | 69.10±1.07 |

Add a small amount of water (0.2 ml water was used for the reconstitution of Formulations 10-11 with 1 ml filling volume, 0.2 ml and 0.25 ml water were used respectively for the reconstitution of Formulation 12 with 1 ml filling volume, 0.25 ml water was used for the reconstitution of Formulation 13 with 1 ml filling volume, 0.55 ml water was used for the reconstitution of Formulations 14-16 with 2.5 ml filling volume) to make the concentration of the monoclonal antibody in the formulation after reconstitution being from about 120 to about 190 mg/ml, and the concentration of the monoclonal antibody obtained, the osmotic pressure and the viscosity are shown in Table 13. The viscosity of each reconstituted formulation is relatively low, and the osmotic pressure is suitable for subcutaneous administration.

**Table 13. Viscosity and osmotic pressure of each reconstituted formulation**

| | Infliximab (mg/ml) | Viscosity (cP) | Osmotic pressure (mOsmol/kg) |
|---|---|---|---|
| Formulation 10 | 155.43 | 21.87 | 390 |
| Formulation 11 | 157.65 | 21.03 | 502 |
| Formulation 12 | 186.11 | 27.04 | 534 |
| (use 0.2 ml for reconstitution) | | | |
| Formulation 12 | 157.72 | 23.56 | 452 |
| (use 0.25 ml for reconstitution) | | | |
| Formulation 13 | 161.03 | 25.17 | 491 |
| (use 0.25 ml for reconstitution) | | | |
| Formulation 14 | 142.15 | 14.48 | 392 |
| Formulation 15 | 165.50 | 28.27 | 419 |
| Formulation 16 | 138.30 | 12.78 | 377 |

### Example 4. Feasibility analysis of preparing high-concentration formulation from the commercial infliximab formulation

In the commercial infliximab lyophilized formulation, each bottle contains infliximab 100 mg, sucrose 500 mg, polysorbate 80 0.5 mg, sodium dihydrogen phosphate 2.2 mg, and disodium hydrogen phosphate 4.86 mg. The primary liquids were prepared according to the ratio, and then lyophilized, and after reconstitution with 10 ml and 2.5 ml of water, respectively, the infliximab concentration, viscosity, and osmotic pressure were measured. As can be seen from Table 14, the protein content is only about 9 mg/ml when using 10 ml of water for reconstitution, and the protein content is increased to 31 mg/ml when using 2.5 ml of water for reconstitution, the viscosity of the solution is still small at this time, but the osmotic pressure (689.5 mOsmol/kg) has far exceeded the range of human body tolerance, indicating that it is not feasible to prepare a high-concentration formulation suitable for subcutaneous administration by the commercial formulation.

**Table 14. Infliximab concentration, viscosity, and osmotic pressure in the formulation after being reconstituted with different volumes of water for the commercial Infliximab formulation (100 mg dosage strength)**

| Dilution volume | Infliximab (mg/ml) | Viscosity (cP) | Osmotic pressure (mOsmol/kg) |
|---|---|---|---|
| 10 ml | 8.91±0.08 | 1.34±0.03 | 156.5±0.5 |
| 2.5 ml | 30.71±2.42 | 4.61±0.04 | 689.5±7.5 |

### Example 5. Preparation of infliximab primary liquids with different pH

Formulations 18-21 were designed and formulated containing different pH (Table 15), respectively.

**Table 15. Infliximab primary liquids with different pH (before lyophilization)**

| Sample | Infliximab (mg/ml) | Histidine buffer (mM) | Polysorbate 80 (mg/ml) | Sucrose (mg/ml) | pH |
|---|---|---|---|---|---|
| Formulation 18 | 50 | 20 | 0.1 | 25 | 4.8 |
| Formulation 19 | 50 | 20 | 0.1 | 25 | 5.3 |
| Formulation 20 | 50 | 20 | 0.1 | 25 | 6.5 |
| Formulation 21 | 50 | 20 | 0.1 | 25 | 8.0 |

### Example 6. Preparation of infliximab primary liquids with different sucrose concentrations

Formulations 22-25 were designed and formulated containing different sucrose concentrations (Table 16), respectively.

**Table 16. Infliximab primary liquids with different concentrations of sucrose (before lyophilization)**

| Sample | Infliximab (mg/ml) | Histidine buffer (mM) | Polysorbate 80 (mg/ml) | Sucrose (mg/ml) | pH |
|---|---|---|---|---|---|
| Formulation 22 | 50 | 5 | 0.1 | 10 | 5.5 |
| Formulation 23 | 50 | 5 | 0.1 | 50 | 5.5 |
| Formulation 24 | 50 | 5 | 0.1 | 75 | 5.5 |
| Formulation 25 | 50 | 5 | 0.1 | 100 | 5.5 |

### Example 7. Preparation of infliximab primary liquids with different concentrations of trehalose

Formulations 26-29 were designed and formulated containing different concentrations of trehalose (Table 17), respectively.

**Table 17. Infliximab primary liquids with different concentrations of trehalose (before lyophilization)**

| Sample | Infliximab (mg/ml) | Histidine buffer (mM) | Polysorbate 80 (mg/ml) | Trehalose (mg/ml) | pH |
|---|---|---|---|---|---|
| Formulation 26 | 50 | 20 | 0.1 | 10 | 5.5 |
| Formulation 27 | 50 | 20 | 0.1 | 50 | 5.5 |
| Formulation 28 | 50 | 20 | 0.1 | 75 | 5.5 |
| Formulation 29 | 50 | 20 | 0.1 | 100 | 5.5 |

Unless otherwise stated, all numbers expressing quantities of ingredients, cell culture, processing conditions, and the like, used in the specification (including the claims) are to be understood as being modified by the term "about" under all conditions. Accordingly, unless indicated to the contrary, the numerical parameters are approximate and may vary depending on the desired characteristics sought to be obtained by the present invention. Unless otherwise stated, the term "at least" preceding a list of elements shall be understood to mean each element in the series. Those skilled in the art will recognize, or be able to devise many equivalents of the specific embodiments of the invention described herein without departing from routine experimentation. The appended claims are intended to cover such equivalents.

Those skilled in the art will appreciate that many modifications and variations of the present invention will be made without departing from the spirit and scope thereof. The specific embodiments described herein are offered by way of examples only and are not intended to be limiting. The true scope and spirit of the invention are shown in the appended claims, and the description and examples are only exemplary.

## Claims

1. A lyophilized formulation comprising infliximab, a histidine buffer system, a structure protectant, and a surfactant, wherein
before lyophilization, in the formulation,
the concentration of infliximab is from 10 to 80 mg/ml,
the concentration of histidine in the histidine buffer system is from 1 to 20 mM,
the concentration of the structure protectant is from 5 to 100 mg/ml, and
the concentration of the surfactant is from 0.001 to 1 mg/ml; and
after reconstitution, the concentration of infliximab in the formulation is from 100 to 200 mg/ml.

2. The lyophilized formulation according to claim 1, **characterized in that**,
before lyophilization, the concentration of infliximab in the formulation is from 25 to 60 mg/ml, preferably from 30 to 50 mg/ml, more preferably 40 mg/ml.

3. The lyophilized formulation according to claim 1 or 2, **characterized in that**,
before lyophilization, the concentration of histidine in the formulation is from 3 to 10 mM, preferably 5 mM.

4. The lyophilized formulation according to any one of claims 1 to 3, **characterized in that**, the structure protectant is selected from the group consisting of carbohydrates, amino acids, and combinations thereof, preferably selected from the group consisting of sucrose, trehalose, and combinations thereof, more preferably sucrose, and
before lyophilization, the concentration of the structure protectant is from 5 to 75 mg/ml, preferably from 5 to 50 mg/ml, more preferably 25 mg/ml.

5. The lyophilized formulation according to any one of claims 1 to 4, **characterized in that**, the surfactant is selected from the group consisting of polysorbates, poloxamer, triton, sodium dodecyl sulfate, sodium lauryl sulfate, sodium octyl glycoside, lauryl-sulfobetaine, polyethylene glycol, polypropylene glycol or their mixtures, preferably polysorbate 80, and before lyophilization, the concentration of the surfactant is from 0.01 to 0.4 mg/ml, preferably from 0.025 to 0.1 mg/ml.

6. The lyophilized formulation according to any one of claims 1 to 5, **characterized in that**, before lyophilization, the pH of the formulation is from 4.0 to 8.0, preferably from 4.5 to 6.0, more preferably from 5.0 to 5.4.

7. The lyophilized formulation according to any one of claims 1 to 6, **characterized in that**, after reconstitution, the formulation has at least one of the following properties:
the viscosity is 40 cP or less, and
the osmotic pressure is from 250 to 530 mOsmol/kg.

8. The lyophilized formulation according to any one of claims 1 to 7, **characterized in that**, after reconstitution, the concentration of infliximab in the formulation is from 130 to 190 mg/ml, more preferably from 140 to 170 mg/ml, from 150 to 160 mg/ml.

9. Use of the lyophilized formulation according to any one of claims 1 to 8 for the manufacture of a medicament for the prevention or treatment of human autoimmune-associated diseases.

10. The use according to claim 9, **characterized in that**,
the diseases comprise rheumatoid arthritis, ankylosing spondylitis, Crohn's disease, ulcerative colitis, psoriatic arthritis and plaque psoriasis.
